(19) Europäisches Patentamt
European Patent Office
Office européen des brevets

(11) **EP 2 637 571 B1**

(12) **EUROPEAN PATENT SPECIFICATION**

(45) Date of publication and mention
of the grant of the patent:
**08.01.2020 Bulletin 2020/02**

(51) Int Cl.:
*A61B 8/14* (2006.01)   *A61N 7/00* (2006.01)
*A61B 8/12* (2006.01)

(21) Application number: **11840646.1**

(22) Date of filing: **11.11.2011**

(86) International application number:
**PCT/US2011/060362**

(87) International publication number:
**WO 2012/065058 (18.05.2012 Gazette 2012/20)**

(54) **REMOTE CENTER OF MOTION ROBOT FOR MEDICAL IMAGE SCANNING AND IMAGE-GUIDED TARGETING**

ROBOTER MIT FERNEM BEWEGUNGSZENTRUM ZUM SCANNEN MEDIZINISCHER BILDER UND FÜR BILDGESTEUERTE ANZIELUNGEN

ROBOT À CENTRE DE MOUVEMENT DISTANT POUR BALAYAGE D'IMAGE MÉDICALE ET CIBLAGE GUIDÉ PAR IMAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.11.2010 US 412589 P**

(43) Date of publication of application:
**18.09.2013 Bulletin 2013/38**

(73) Proprietor: **The Johns Hopkins University Baltimore, MD 21218 (US)**

(72) Inventors:
• **STOIANOVICI, Dan**
**Reisterstown, Maryland 21136 (US)**
• **PETRISOR, Doru**
**Towson, Maryland 21286 (US)**
• **SCHAFER, Felix**
**Baltimore, Maryland 21224 (US)**
• **KIM, Chunwoo**
**Baltimore, Maryland 21224 (US)**
• **HAN, Misop**
**West Friendship, Maryland 21794 (US)**

(74) Representative: **Murgitroyd & Company**
**Murgitroyd House**
**165-169 Scotland Street**
**Glasgow G5 8PL (GB)**

(56) References cited:
US-A- 5 820 623        US-A1- 2005 261 591
US-A1- 2007 021 738    US-A1- 2008 249 403
US-A- 2008 314 181     US-A1- 2009 234 369
US-A1- 2009 234 369    US-A1- 2009 248 038
US-A- 2010 056 900

**Description**

**STATEMENT OF GOVERNMENTAL INTEREST**

This invention was made with U.S. government support under grant no. 1R21CA141835-01. The U.S. government has certain rights in the invention.

**FIELD OF THE INVENTION**

The present invention pertains to a remote center of motion robot for medical image scanning. More particularly, the present invention pertains to a remote center of motion robot for medical image scanning and image-guided targeting.

**BACKGROUND OF THE INVENTION**

Prostate cancer is the most common form of cancer in American men. Like other cancers, early diagnosis and treatment is critical to the clinical management of the disease, and to preserving patients' quality of life and increasing life expectancy. In furtherance of these objectives, advanced imaging technologies have been developed to improve physicians' ability to accurately detect and stage clinically relevant cellular changes and to deliver treatment.

Ultrasound elastography is an emerging imaging modality with a potential to improve cancer detection. The principle behind elastography is that different tissues exhibit distinct strain profiles under stress. If stress is induced (excited) with an ultrasound probe, images may be used to visualize the resulting strain patterns. During compression and retraction motions, zones of the image that remain relatively uncompressed reveal zones of higher stiffness which may correlate to cancer nodules. Elastography currently involves freehand probe motion to induce the stress. Consequently, the uniformity of repeated compression and retraction of the probe is critical to optimizing the reliability of elastography.

Physicians typically screen for prostate cancer by performing a digital rectal examination and measuring the level of Prostate Specific Antigen (PSA) in the blood. Following an abnormal rectal exam or finding of elevated PSA levels, physicians confirm the presence of pathological tissue by taking a biopsy of the prostate. Prostate biopsy may be performed either transrectally (TR), during which a biopsy needle is inserted through the wall of the rectum, or via the transperineal (TP) route whereby physicians insert needles percutaneously in the region between the scrotum and anus. In either case, appropriate needle insertion sites are determined based on physician experience and the procedure is guided by transrectal sonography or another imaging technology.

Multiple treatments methods are available to patients whose biopsy results show abnormal histological changes within the prostate. For several decades, the definitive treatment for low and medium risk prostate cancer was radical prostatectomy or external beam radiation therapy. More recently, practitioners have successfully used brachytherapy to achieve equivalent outcomes. Brachytherapy accounts for a significant and growing proportion of prostate cancer treatments, as it is delivered with minimal invasiveness in an outpatient setting. Brachytherapy involves inserting multiple radioactive "seeds" into the prostate gland either temporarily (high dose-rate (HDR) brachytherapy), or permanently (low dose rate (LDR) brachytherapy). Brachytherapy, like transrectal and transperineal biopsy, requires multiple punctures to obtain multiple tissue cores. Transperineal biopsy is often performing using brachytherapy mapping templates to facilitate localization of cancerous tissue and to guide future biopsies. Both brachytherapy and biopsy are typically guided by 2-dimensional (2-D) transrectal ultrasound (TRUS). While 2-D TRUS provides adequate imaging of the soft tissue anatomy, it does not allow for localization or precise placement of biopsy needles or implanted brachytherapy seeds.

Another available treatment option for prostate cancer is laparoscopic radical prostatectomy (LRP). During prostatectomy, precise resection of the tumor-containing prostate gland and the preservation of neighboring anatomical structures are critical to preventing tumor recurrence and incontinence, and to preserving sexual potency. However, visualization of anatomical structures adjacent to the prostate can be challenging due to periprostatic connective tissues and intraoperative hemorrhage, even when the site is viewed with surgical loupes during open surgery or under laparoscopic magnification. To solve this problem, surgeons have used TRUS during LRP and have found that TRUS images may provide a decreased rate of positive surgical margins and may help preserve neighboring structures. However, using TRUS to guide LRP has posed several challenges. First, TRUS probes are often operated manually; as such, image stability is comprised and the probe position data that is required for 3-D computation is lost. Also, TRUS systems have been too large to be used in tandem with advanced robotically-assisted minimally invasive surgical systems, where the space between the surgical robot and the patient is too limited to accommodate a human assistant for operating the TRUS system.

Robotic systems have recently emerged for TRUS probe tracking and needle interventions. Although these systems improve image stability and allow the operator to track probe position, these systems typically use extrinsic optical or electromagnetic tracking systems that lack the automated motion capabilities required to obtain uniform images or to digitally lock a trajectory. Another limitation of some robotic systems is that they commonly handle the needle rather than the probe and are built for transperineal rather than endocavity access. Therefore, the systems are not adaptable for TRUS-guided LRP and the points at which brachytherapy or biopsy needles are inserted must still be determined based on surgeon experience.

Accordingly, there is a need in the art for a robotic ultrasound manipulator that has a more compact configuration to facilitate brachytherapy, needle biopsy, image-guided LRP, and ultrasound elastography, and that allows practitioners to visualize a 3-D reconstruction of an area under cancer treatment.

US Patent Application Publication No. US 2009/0234369 A1 describes a guide apparatus for orienting a medical tool relative to and through a remote fulcrum or remote center of motion. The apparatus comprises at least one crank arm comprising at least a portion of a first hinged coupling for hinged coupling to a stabilizer; at least one link arm comprising at least a portion of a second hinged coupling for hinged coupling to the crank arm at a location spaced from the first hinged coupling; a tool holder for supporting a medical tool on the link arm at a location spaced from the first hinged coupling; wherein the rotational axes of the first and second hinged couplings intersect to define a remote fulcrum.

## SUMMARY

The invention is defined by independent claim 1. Further embodiments of the invention are defined by the dependent claims.

## BRIEF DESCRIPTION OF THE DRAWINGS

The accompanying drawings provide visual representations which will be used to more fully describe the representative embodiments disclosed herein and can be used by those skilled in the art to better understand them and their inherent advantages. In these drawings, like reference numerals identify corresponding elements and:

FIG. 1 illustrates a kinematic diagram of an exemplary device according to the features of the present invention.
FIG. 2 illustrates a kinematic diagram of another exemplary device according to the features of the present disclosure.
FIG. 3(a) illustrates a graphical representation of the RCM mechanism implementing Z-Y-X Euler angles according to the features of the present invention.
FIG. 3(b) illustrates a graphical representation of the fixed frame X-Y-Z rotations of the tool frame {T} in the fixed base frame {B} according to the features of the present invention.
FIG. 4 illustrates a perspective view of portions of the exemplary device according to features of the present invention.
FIG. 5 illustrates a side elevational view of portions of the exemplary device according to features of the present invention.
FIG. 6 illustrates an exploded view of a driver module of the exemplary device according to features of the present invention.
FIG. 7 illustrates a base holder with an encoder for the driver module of the exemplary device according to FIG. 6.
FIG. 8 illustrates a perspective view of yet another exemplary device according to features of the present disclosure.
FIG. 9 illustrates a side elevation view of the yet another exemplary device according to features of the present disclosure.
FIG. 10 illustrates an exploded view of a driver module of the yet another exemplary device according to features of the present disclosure.
FIG. 11 illustrates a schematic of the exemplary device used in tandem with a DA VINCI® robot according to features of the present invention.
FIG. 12 illustrates a schematic view of the exemplary device used in tandem with a DA VINCI® robot according to features of the present invention.
FIG. 13 illustrates a system block diagram and safety features of the exemplary system according to features of the present invention.
FIG. 14(a) is a photograph of the experimental setup having a TRUS probe supported by the exemplary device according to features of the present invention.
FIG. 14(b) is a post-processed 3-D reconstruction from ultrasound images based on segmentation according to features of the present invention.
FIG. 14(c) is a fast processed 3-D representation of the imaged gland using volume rendering of the gathered image space.

## DETAILED DESCRIPTION OF THE PREFERRED EMBODIMENTS

The presently disclosed subject matter now will be described more fully hereinafter with reference to the accompanying Drawings, in which some, but not all embodiments of the inventions are shown. Like numbers refer to like elements throughout. The presently disclosed subject matter may be embodied in many different forms and should not be construed as limited to the embodiments set forth herein; rather, these embodiments are provided so that this disclosure will satisfy applicable legal requirements. Indeed, many modifications and other embodiments of the presently disclosed subject matter set forth herein will come to mind to one skilled in the art to which the presently disclosed subject matter pertains having the benefit of the teachings presented in the foregoing descriptions and the associated Drawings. Therefore, it is to be understood that the presently disclosed subject matter is not to be limited to the specific embodiments disclosed.

The present invention pertains to a remote center of motion robot for medical image scanning and image-guided targeting, hereinafter referred to as the "Euler" robot. The Euler robot allows for ultrasound scanning for 3-Dimensional (3-D) image reconstruction and enables a variety of robot-assisted image-guided procedures, such as needle biopsy, percutaneous therapy delivery, image-guided navigation, and facilitates image-fusion with other imaging modalities. The Euler robot can also be used with other handheld medical imaging probes, such as gamma cameras for nuclear imaging, or for targeted delivery of therapy such as high-intensity focused ultrasound (HIFU). 3-D ultrasound probes may also be used with the Euler robot to provide automated image-based targeting for biopsy or therapy delivery. In addition, the Euler robot enables the application of special motion-based imaging modalities, such as ultrasound elastography. The Euler robot uses remote center of motion kinematics, which is a characteristic of minimally invasive access devices. As such, the Euler robot according to features of the present invention is also applicable to manipulating other medical instruments and devices, such as laparoscopic instruments, including ultrasound.

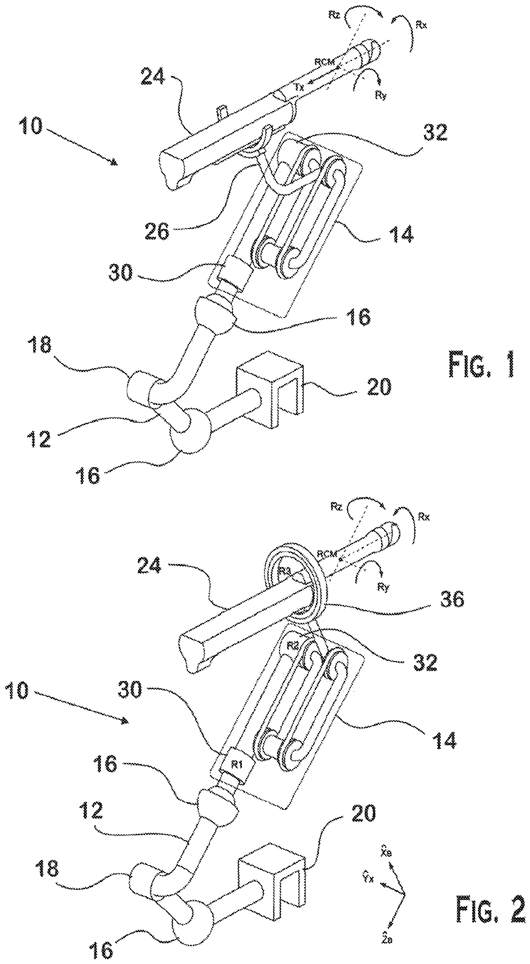

With reference to FIGS. 1 and 2, exemplary embodiments of the Euler robot 10 are illustrated. The Euler robot 10 includes a support arm 12 and a remote center of motion (RCM) module 14, which is operatively connected to the support arm 12. The support arm 12 is shown as having a generic structure with two spherical joints 16 and one cylindrical joint 18 which can be located at a desired location to support the base of the Euler robot 10 in place as needed for the clinical operation. However, various other structures are possible, depending upon application and design preference. A mount 20 may also be provided at the end of the support arm 12, which can be attached to a fixed support such as a medical table.

The remote center of motion module preferably has a parallelogram structure built with belts, as described in U.S. Patent No. 7,021,173 (the '173 patent). As discussed in the '173 patent, the RCM module includes first, second, and third connecting link units, the first connecting link unit coupled to a base link unit at a passive revolute joint. The base adjustment angle can be changed by adjusting the angle between the base link unit and first connecting link unit. The base link unit further comprises a base shaft, which provides one rotational degree of freedom about a first axis. A second connecting link unit is coupled to the first connecting link unit at a revolute joint, and the second connecting link unit is further coupled to a third connecting link unit by another revolute joint. The third connecting link unit is configured to receive, at another joint (output shaft), a driver or holder for an end-effector, such as an ultrasound probe, biopsy needle, or other medical instrument.

As further described in the '173 patent, the revolute joint between the first and second connecting link units may be actuated by motor, while the revolute joints between the second and third connecting link units and at the output shaft are coupled to the first revolute joint through a belt-drive mechanism. Thus, the second rotational axis of the RMC module that provides a second degree of freedom is materialized by the first, second, and third connecting link units and the end-effector/driver holder. The system has been designed so that the second connecting link may be actuated with respect to the first, while the third connecting link maintains its parallel orientation with respect to the first connecting link. The end-effector holder maintains its parallel orientation with respect to the second connecting unit. The configuration of the first and second rotation axes and corresponding rotating joints and connecting links form a double-parallelogram-based structure. This design allows for rotating the end-effector about an axis, y, which is remote from the mechanism and forms the classic RCM design.

With reference back to FIGS. 1 and 2, the RCM module 14 presents two rotary degrees of freedom (DOF) (Ry, Rz) about joints 30 and 32 with axes intersecting at an RCM pivot point. An important feature of this RCM mechanism is that its structure is located from the pivot point, so that space is cleared from the medical intervention and current instrument and/or probe. However, it should be understood that other types of RCM mechanism may be used, depending upon application and design preference.

With reference in particular to FIG. 1, a driver module 26 (RT driver) is operatively connected to the RCM module 14. The driver module manipulates an imaging probe 24 positioned therein in at least two degree of freedoms. For example, the driver module 26 may provide one degree of freedom around a first rotation axis Rx and one linear degree of freedom for translation along a second axis Tx.

With reference to FIG. 2, an alternative driver module 36 (R driver) may be operatively connected to the RCM module 14. The driver module 36 manipulates the imaging probe 24 (positioned therein) in one degree of freedom. Preferably, the driver module 36 provides one degree of freedom around a first rotation axis Rx. However, it should be understood that other type of driver modules are possible, depending upon application and design preference. In particular, the driver modules can be constructed such that palpations are allowed for elastography and/or the robot is adapted for holding and operating a needle for biopsy.

According to the Euler robot 10, the probe 24 is moved to sweep the region of interest for imaging. Motion is also used to orient the probe as needed for image-guided targeting. A main advantage is that the robot automatically tracks the position and orientation of the image space because it handles the probe. As such, intraoperative image-to-robot registration which is common for most image-guided robots is not required. Instead, a calibration is performed once, ahead of time, and should remain constant.

With classic 3-D ultrasound probes the location of imaged 3-D volumes relative to the probe changes with the motion of the probe. This makes it difficult to follow a precise anatomical target within the continuously changing image. With the Euler robot 10, target location does not change in the fixed robot frame and the robot may automatically position the probe 24 to show it in the image. Digital targeting (aiming a target defined by its image coordinates) is facilitated by the use of the Euler robot 10. In addition, the Euler robot 10 enables 3-D imaging with less expensive, higher image quality 2-D equipment.

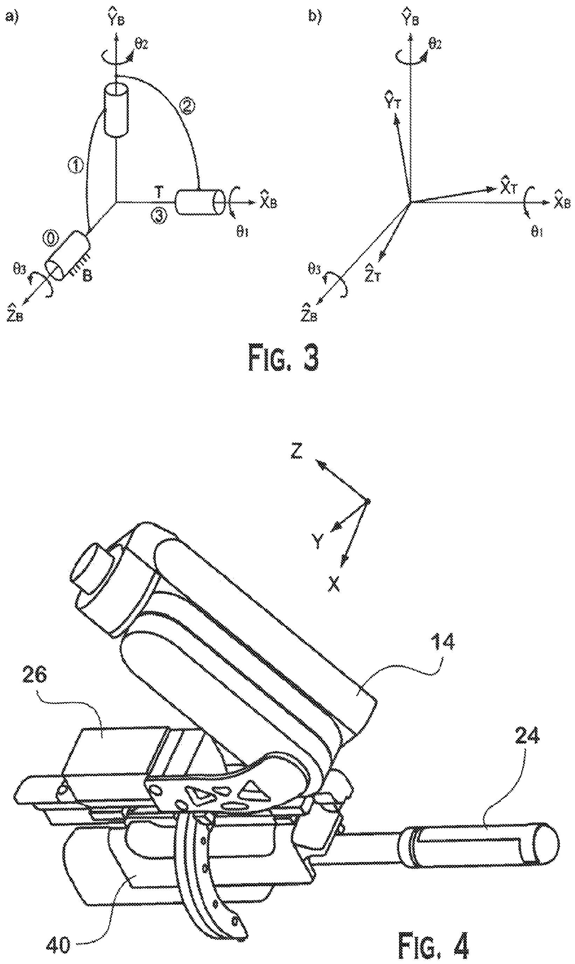

With reference to FIGS. 3(a) and (b), the orientation of the tool frame T (end-effector instrument) can be described by a classic set of yaw, pitch, and roll Euler angles (Z-Y-X). In particular, FIG. 3(a) is a symbolic representation of the RCM module implementing Z-Y-X Euler angles, and FIG. 3(b) is a fixed frame X-Y-Z rotations of its tool frame {T} in the fixed base frame {B}.

Preferably, the remote axis of the RCM mechanism (Ry) uses a parallelogram mechanism implemented with two belts, as described in U.S. Patent No. 7,021,173. Since these joints are mechanically coupled, they are represented symbolically with a single joint connecting links 1-2. The Euler robot implements a sequence of three rotations ($\Theta_3$, $\Theta_2$, $\Theta_1$) performed about the axes of the moving frames, starting from the base frame {B} of link (0) to the tool frame {T} of link (3).

This RCM3 link configuration has a very interesting robot kinematic consequence, as follows. The Euler $\hat{Z} \rightarrow \hat{Y} \rightarrow \hat{X}$ rotation about the axes of the moving frames of the manipulator can be expressed as:

$$
{}^B_T R = {}^0_3 R = {}^0_1 R \; {}^1_2 R \; {}^2_3 R = R_z(\Theta_3) \; R_y(\Theta_2) \; R_x(\Theta_1) \qquad \text{(Eq. 1)}
$$

where R represent the rotation matrices of the individual frames associated with the links. The other way, a tool frame orientation can be expressed as a sequence of rotations about the $\hat{X}_B \rightarrow \hat{Y}_B \rightarrow \hat{Z}_B$ axes of the fixed {B} coordinate frame shown in b. These three fixed frame sequential rotations move an arbitrary point ${}^B P$ defined in the base frame {B} as follows:

$$
\begin{aligned}
\Theta_1: & & {}^B P \rightarrow & & R_x(\Theta_1) \; {}^B P \\
\Theta_2: & & R_x(\Theta_1) \; {}^B P \rightarrow & & R_y(\Theta_2) R_x(\Theta_1) \; {}^B P \\
\Theta_3: & R_y(\Theta_2) R_x(\Theta_1) \; {}^B P \rightarrow & & R_z(\Theta_3) R_y(\Theta_2) R_x(\Theta_1) \; {}^B P
\end{aligned} \qquad \text{(Eq. 2)}
$$

so that the overall rotation is:

$$
R_{xyz} = R_z(\Theta_3) \; R_y(\Theta_2) \; R_x(\Theta_1) \qquad \text{(Eq. 3)}
$$

Since individual axis rotation matrices $R_x$, $R_y$, $R_z$ in (1) and (3) are the same, it results that ${}^B_T R = R_{xyz}$ , which shows that fixed X-Y-Z rotations and Euler Z-Y-X yield the same final orientation. The kinematic implication of using this mechanism and rotation frames is that the direct and inverse kinematic solutions of the 3-RCM mechanism are trivial: RCM joint angles $\Theta_1$, $\Theta_2$, $\Theta_3$ equal their respective tool frame angles $\Theta_1$, $\Theta_2$, $\Theta_3$ about the fixed axes of the base coordinate system, and vice-versa.

When the tool frame orientation is given by means other than fixed frame rotations, these can easily be derived in a closed form. For example, from (1) the direct kinematic solution to a tool frame described by a common rotation matrix is:

$$\begin{aligned}
{}_T^B R &= \begin{bmatrix} c3 & -s3 & 0 \\ s3 & c3 & 0 \\ 0 & 0 & 1 \end{bmatrix} \begin{bmatrix} c2 & 0 & s2 \\ 0 & 1 & 0 \\ -s2 & 0 & c2 \end{bmatrix} \begin{bmatrix} 1 & 0 & 0 \\ 0 & c1 & -s1 \\ 0 & s1 & c1 \end{bmatrix} = \\[2mm]
&\begin{bmatrix} c2c3 & s1s2c3 - c1s3 & c1s2c3 + s1s3 \\ c2s3 & s1s2s3 + c1c3 & c1s2s3 - s1c3 \\ -s2 & s1c2 & c1c2 \end{bmatrix} \quad \text{(Eq. 4)}
\end{aligned}$$

where $sn = \sin\Theta_n$ and $cn = \cos\Theta_n$, for $n = 1,2,3$.

For the inverse, the elements $r$ of the tool frame rotation matrix are given:

$$\begin{matrix} {}_T^B R = \begin{bmatrix} r_{11} & r_{12} & r_{13} \\ r_{21} & r_{22} & r_{23} \\ r_{31} & r_{32} & r_{33} \end{bmatrix} & \text{(Eq. 5)} \end{matrix}$$

RCM joint angles are then simply derived observing that $r_{11}^2 + r_{21}^2 = c2^2$. When $c2 \neq 0$:

$$\begin{aligned}
\Theta_2 &= \tan^{-1} \frac{-r_{31}}{\sqrt{r_{11}^2 + r_{21}^2}} \\[2mm]
\Theta_3 &= \tan^{-1} \frac{r_{21}}{r_{11}} \qquad \text{(Eq. 6)} \\[2mm]
\Theta_1 &= \tan^{-1} \frac{r_{32}}{r_{33}}
\end{aligned}$$

This also describes the singularity of the RCM3 mechanism for $\neq 0$, where this loses a degree of freedom when its $\hat{X}$ and $\hat{Z}$ axes are aligned. In practice this is not typically a problem because this alignment is prevented by other mechanism constraints and patient interference, as shown in the following sections. The R and RT Drivers are also built with an offset of the $\Theta_2$ angle, of $\Theta_2^0 = 35°$ and $\Theta_2^0 = -30°$ respectively, to increase the clearance of the mechanism with respect to the imaging site and patient.

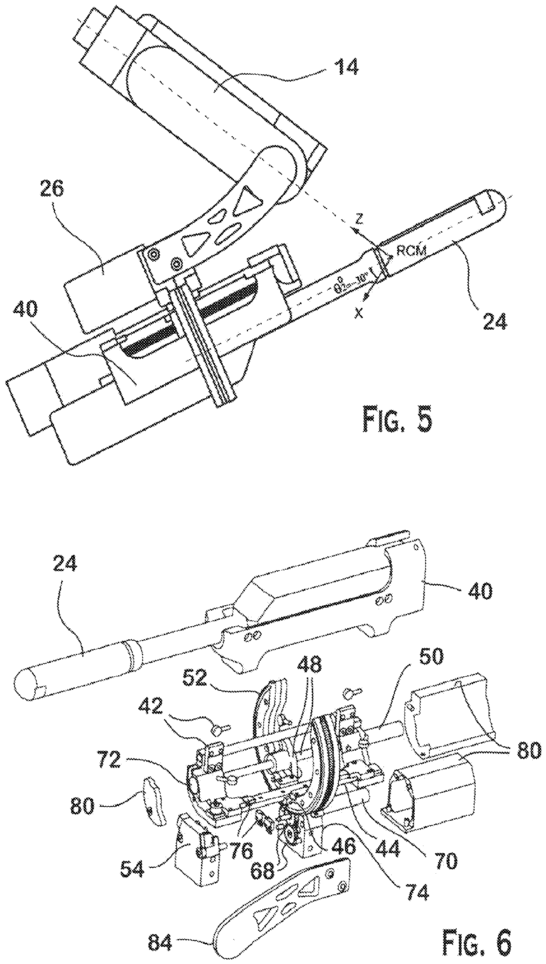

With reference to FIGS. 4-7, the Euler robot with the driver module 26 (RT driver) will be described in more detail. In particular, the RCM module 14 supporting the ultrasound probe 24 is illustrated in its folded/collapsed position, which the parallelogram is collapsed, as shown in FIGS. 4 and 5. An ultrasound probe 24 is mounted using an adapter 40 that is custom built to match the shape of the probe. However, it should be understood that various other probes may be attached to the driver by other special adapters and the like.

With reference to FIG.6, the features of the driver module 26 will be described in more detail. In particular, the adapter 40 may be attached to the driver 26 through preload plates 42, including sensors, as is supported by a structure including a linear rail 44 and guide 46. The linear rail 44 is actuated with a ball screw 48 by a geared servomotor 50. The linear rail 44 and guide 46 are then supported by the rail structure of a custom built rotary rail 52 and guide 54. The rotary rail 52 preferably presents a horseshoe shape and comprises a spur gear sector 56 mounted between two rail parts.

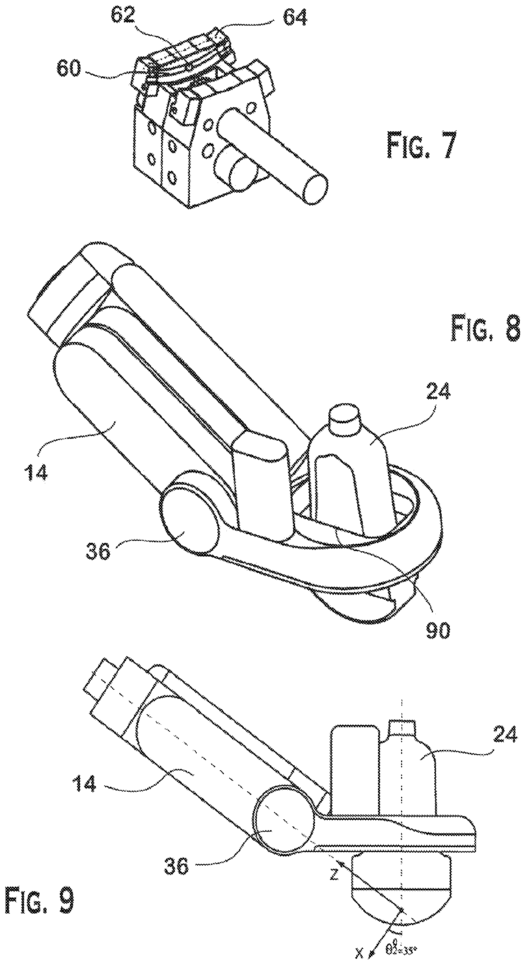

The rotary rail 52 and guide 54 present a similar structure to classic linear ball rails, but the rails have circular geometry so that the resulting motion is rotary rather than linear. As shown in FIG. 7, ball grooves 60 are made in the guides and on each side of the rail 52, and two series of steel balls 62 circulate in these grooves and then recirculate through special paths made within the blocks of the guides through a ball recirculation path 64.

With reference back to FIG. 7, actuation of the horseshoe shaped rotary rail 52 is given by its gear sector 66 which is engaged by a pinion 68 from a geared servomotor 70. Both axes are equipped with redundant encoders 72, 74, one encoder 72 connected directly to the screw 48, the other encoder 74 connected to the pinion 68 of the rotary axes through another pinion. A set of hardware limit switches 76 is used on each axis. Since both the motor and redundant encoders are incremental, these are also used for homing.

Preferably, a set of five force sensors are used to measure all force-torques interactions of the probe 24 except for the torque exerted about its longitudinal axis. Preferably, the force sensors are miniature quartz sensor series with extremely flat design for measuring dynamic and quasistatic forces, but other types of sensors are possible. Four of the sensors are intercalated between the probe adapter 40 and the RT driver 26, to measure all forces and moments exerted lateral to the probe 24. The axial force is measured by a sensor intercalated between the ball nut and the rotary rail 52.

Several caps 80 are used to encase the motors, encoders, and electrical connections. An arm 84 connects the base of the mechanism, which is the rotary guide assembly, to the distal RCM mount (not shown).

As described above, the RT driver 26 implements a rotation which in addition to the RCM module completes the set of three Euler orientation angles. Probe adapters are built so that these align with the longitudinal axis of the probe. The RT driver also provides a linear degree of freedom for motion along this axis. The horseshoe geometry of the rotary rail has been chosen to clear the space above the ultrasound probe and so facilitate manual access and manipulation of needles about the probe for ultrasound-guided interventions such as biopsy. Alternatively, if this access to the probe is not required, the construction can be substantially simplified by using a disk like structure for the rotary axis which eliminates the need for the rotary ball rails and guide.

This specific kinematic arrangement and order of the R and T guides and rails presented above has been chosen based on two considerations: 1) to ensure that the space cleared for manual access remains above the probe during rotation, and 2) to render a compact and sturdy mechanical structure. Since the probe adapter must have a long shape to firmly hold the handle of the probe, the most appropriate place of the linear rail resides along its side, as shown in. The linear guide must then attach to the horseshoe rotary rail. Consequently, the rotary guide should preferably base the driver. However, other structures and orientations are possible, depending upon application and design preference.

With reference to FIGS. 8-10, the Euler robot with the driver module 36 (R driver) will be described in more detail. In particular, the RCM module 14 supporting the ultrasound probe 24 is illustrated in its folded/collapsed position, which the parallelogram is collapsed, as shown in FIGS. 8 and 9. An ultrasound probe 24 is mounted using an adapter 90 that is custom built to match the shape of the probe 24. However, it should be understood that various other probes may be attached to the driver by other special adapters and the like.

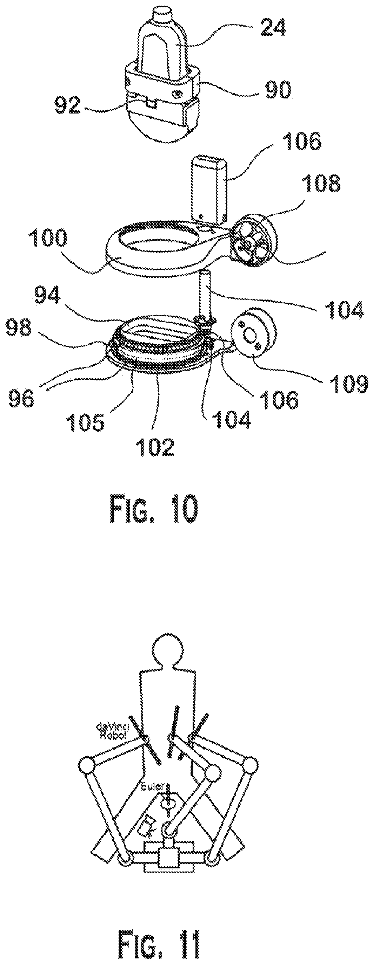

With reference to FIG. 10, an ultrasound probe 24 (external body probe shown) is supported with an adapter 90 so that various probes can be used. The adapter 90 also provides a regular geometric shape for mounting the probe 24 because typical handheld probes have notoriously irregular geometry. The adapter 90 may also include a set of mounting clips 92 for easy attachment/disconnection to the driver 36. Overall, these features ensure that the same probe 24 can be repeatedly connected to the driver 36 in the same relative position and orientation. This is.important to maintain the calibration of the image frame with respect to the robot.

The driver 36 presents a disk like structure. The adapter 90 of the probe 24 connects to a central rotor 94 that spins the probe 24. The rotor 94 is supported by a set of ball bearings 96 and o-rings 98, which help to insolate the mechanisms. To reduce the size of the driver 36, the races of the bearings are built within the body 100 and cap 102 of the driver 36. For simplicity, the bearings do not use a cage, but a sequence of intercalated slightly smaller balls. These normally roll in a reversed direction than the main balls possibly reducing friction. The rotor 94 is engaged by a spur gear transmission from a servomotor 104 having gears 105, which is contained within the motor cover 106. A limit switch 104 mounted on the base triggers a rotary location for homing an incremental encoder. The driver 36 does not use redundant encoding, but could simply be added with another pinion 107 engaged by the gear, similar to the RT Driver described above.

The body 100 of the driver connects to the RCM module 14 through a custom made force sensor 108 to measure the force exerted on the probe in the axial direction, which is secured within the mount caps 109. A structure of four strain concentration elements may be used. Strain gauges are applied to the opposite sides of a bridge and a half-bridge connection may be used for the measurement.

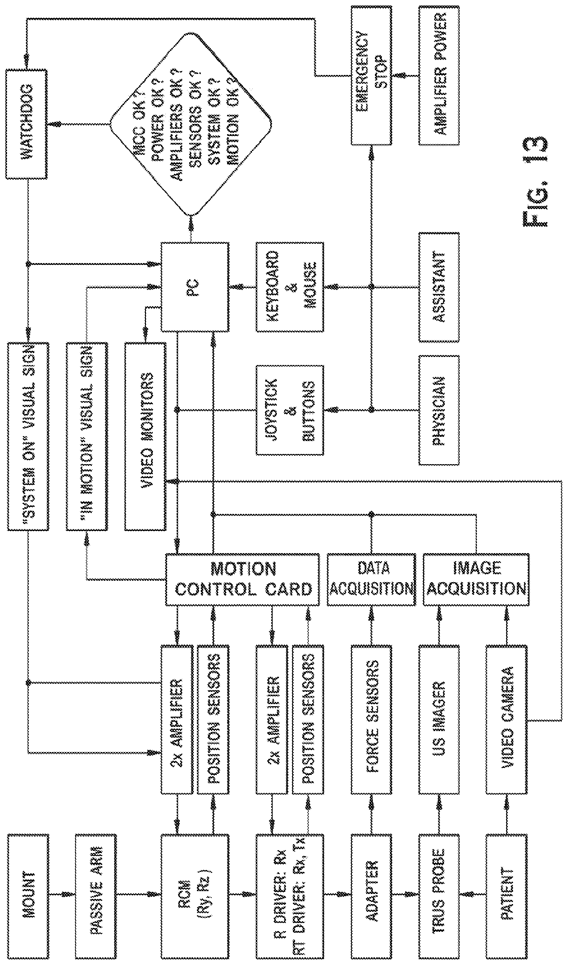

With reference to FIG. 13, a block diagram of the system is presented in, showing its operation with an ultrasound probe. A video camera is also included for system monitoring in applications where direct observation of the Euler robot may be difficult, such as the tandem robot approach described in the following sections.

The controller of the robot may be based on an Intel (Santa Clara, CA), Core 2 Quad Q9550 processor on Asus (ASUSTeK Computer Inc., Taiwan), P5N7A-VM motherboard with 4GB of DDR2 memory running Windows XP (Microsoft Corp, Redmond, WA). The computer system may be equipped with a built-in uninterruptable power supply or operation safety and short range portability. Motor control may be implemented on an MC8000-DUAL (PMDI, Victoria, B.C., Canada) motion control card with onboard digital signal processor for real-time motion control. This card presents dual quadrature decoders and counters for each axis, used for the motor and redundant encoders of each axis. Linear servomotor amplifiers may be used for all four axes. A purpose built relay and watchdog board may be included. A single connector cable connects the PC to a connection box at the base of the passive arm. The arm mounts to a custom oversized rail for increased rigidity. The connection box also includes the amplifiers for force sensors to reduce the length of force sensor cables.

The watchdog checks the state of several components of the system once every 100ms, disabling power to the motor amplifiers if a faulty condition is detected. Two visual signs are used to signal the operation state of the robot being powered and in motion. An Emergency Stop button disables the system and also suspends motor amplifier power. The operation of the system is performed from either a 2-DOF joystick equipped with RCM or RT Driver enable buttons, or under numeric control.

The software of the TRUS Robot consists of axis-level motion control software, robot kinematics software, ultrasound visualization, and 3-D image processing software. Motion control software is built in Visual C++ (Microsoft Corp, Redmond, WA) based on high-level libraries of functions of the motion control card (MCI-SoftLib, PMDI). Imaging components and interface to the robot are also written in Visual C++ based on the Amira Visualization platform (Visage Imaging Inc, San Diego, CA).

When a probe is manipulated by the TRUS Robot, the positional data along with the images is readily available. This allows recording image-position data pairs for robot navigation and 3-D reconstruction. The robot may scan the prostate in arbitrary directions. A rotary scan about the probe axis is normally proffered to reduce soft tissue deflections. Special imaging algorithms were developed for the rotary scan, because typically 3-D reconstruction is based on parallel image slices (for example CT or MRI).

Accordingly, the present robot facilitates navigation during surgery and medical interventions. The robotic apparatus may be used to manipulate the ultrasound imaging transducer, in tandem with the at least one medical instrument suitable as a fiducial marker. A region of interest is scanned and at least one parameter of at least one anatomical feature is measured therein with the ultrasound imaging transducer. The position of the imaging transducer is tracked during surgery and interventions using a programmable computer linked to the robotic apparatus. The information obtained from tracking is applied to the transducer to construct at least one three-dimensional model of the region of interest in which the medical instrument can be visualized during the surgery or intervention. The medical instrument is manipulated about the region of interest using the information derived from the at least one three-dimensional model.

The particular medical instrument used preferably includes at least one arm and associated or incorporated instrument of a robotically-assisted minimally invasive surgical system, including a therapy delivery device, needle for obtaining tissue samples, fiducial marker, or laparoscopic or other surgical instrument. The robotic apparatus may be used in tandem with a medical procedure performed manually or robotically. During scanning of the ultrasound transducer, the medical instrument is visible as a hyperechoic region in at least one live ultrasound image captured intraoperatively. The apparatus manipulates the ultrasound imaging transducer to scan in arbitrary directions. The robotic apparatus allows continuous tracking of the ultrasound traducer position within the available reference frame. A computer system captures at least one ultrasound image and the corresponding ultrasound imaging transducer position coordinates. The robotic apparatus provides measurements of the remote center of motion and ultrasound transducer drive angles from which ultrasound images are acquired. The information gathered from the images are segmented and used for producing a three-dimensional ultrasound image volume of anatomic features of interest in the target surgical site.

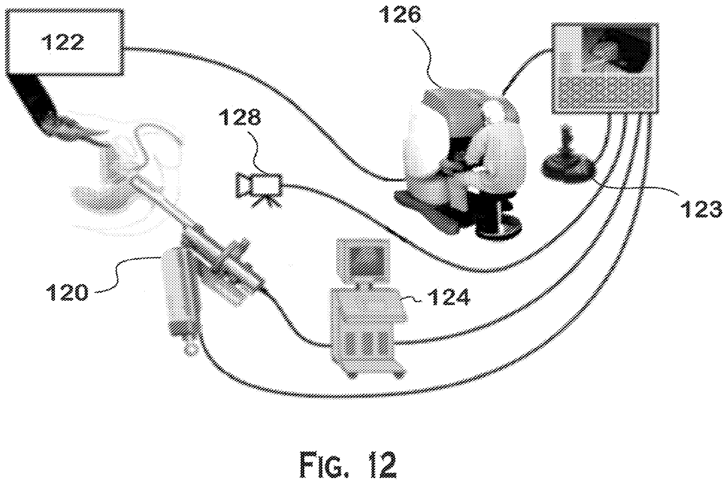

With reference to FIGS. 11 and 12, the Euler robot 120 is used in tandem with a DA VINCI® robot 122. The Euler robot 120 may be attached to the end of the surgical table, in the confined space below the DA VINCI® robot 122, and may be maneuvered by a joystick 123. As shown in FIG. 12, ultrasound images are acquired through a computer system 124 and presented to the surgeon on the console 126 along the current laparoscopic view using a side-by-side display. When using the DA VINCI® robot 122, a video camera 128 may be used to visualize the surgical site, and provide images to the display.

As discussed above, the Euler robot may be used in connection with elastography. In particular, a programmable computer system may be provided for controlling repeated compression and retraction of the ultrasound transducer for palpating the anatomic region of interest. Similarly, the Euler robot may be used in connection with performing needle biopsies on a patient. In particular, a programmable computer system may be provided for controlling insertion and retraction of the biopsy needle to obtain a tissue sample from an anatomic region of interest.

## EXAMPLE

The following Example has been included to provide guidance to one of ordinary skill in the art for practicing representative embodiments of the presently disclosed subject matter. In light of the present disclosure and the general level of skill in the art, those of skill can appreciate that the following Example is intended to be exemplary only and that numerous changes, modifications, and alterations can be employed without departing from the scope of the presently disclosed subject matter. The following Example is offered by way of illustration and not by way of limitation.

Two types of prototypes were built in the laboratory- the Euler-R (corresponding to robot with driver module 36) and Euler-RT (corresponding to robot with driver module 26). Both versions presented a compact structure and, due to their RCM kinematic architecture, have a wide range of motion which enables typical positioning and scanning motion (Table 1) that is similar to human maneuverability of a probe. These values are relative to the folded/collapsed position of the RCM module, ( $\Theta_2^0 = 35°$ for Euler-R and $\Theta_2^0 = -30°$ for Euler-RT). The effective $\Theta_2$ limit angles in the table show that the operation of the RCM3 mechanism is not in the vicinity of singular positions described in (5-6). Software limits within the hard limits are also imposed as clinically required and for safety considerations.

Table 1: Hardware limits of the Euler robots

| | Rx | Ry | | | Rz | Tx |
|---|---|---|---|---|---|---|
| | $\theta_1$ [°] | Constructive Offset $\theta_2^0$ [°] | $\theta_2$ [°] when collapsed | Effective $\theta_2$ [°] | $\theta_3$ [°] | \|mm\| |
| Euler R | Unrestricted | 35° | -19° to +90° | -55° to +54° | ±85° | N/A |
| Euler RT | ±115° | -30° | -18° to +45° | -48° to +15° | ±85° | ±34 |

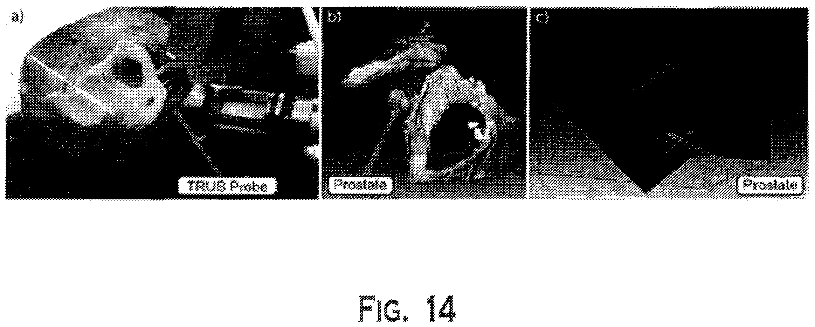

Several pelvic models were built. FIG. 14(a) shows a mockup which simulates the pelvic bones, prostate, bladder, urethra, and two structures representing neurovascular bundles. The mockup was gelatin based, made of 300 Bloom gelatin powder in solution with sorbitol, glycerin, and water, to create a realistic ultrasound view. Images acquired with a rotary scan were post processed for segmentation and represented, as shown in FIG. 14(b). FIG. 14(c) shows a 3-D representation of the prostate that is generated directly from the "gathered" image space, using a "volume rendering" mode. This can display the 3-D shape of the gland without segmenting it. Volume rendering displayed each gathered image voxel translucent, and light absorption through the voxel was correlated to its brightness according to a scale factor.

Accordingly, the robotically-assisted ultrasound manipulator of the present invention allows intrinsic tracking of the probe position and the orientation of the image space, which greatly benefits performing brachytherapy, needle biopsy, LRP, and transrectal ultrasound elastography. Practitioners also benefit from the remote center of motion kinematics in a compact system that extends the application range to manipulating medical instruments and devices such as laparoscopic instruments, including laparoscopic ultrasound. Also, the compact configuration allows practitioners to use the robot in tandem with robotically-assisted minimally invasive surgical systems. Finally, a robotic system that automatically tracks ultrasound probe position and that permits the uniform application and retraction of the probe optimizes the quality and reliability of ultrasound elastography.

**Claims**

1. An apparatus for supporting and manipulating an imaging probe (24) in performing ultrasound-guided interventions, comprising:

   a remote center of motion module (14) providing to an imaging probe (24) at least a first rotational degree of freedom around a first axis (Ry) and a second rotational degree of freedom around a second axis (Rz); and
   a driver module (26) operatively connected to the remote center of motion module (14), the driver module (26) providing a linear degree of freedom for translation along an axis (Tx) aligned with a longitudinal axis of the imaging probe (24) and further providing a third rotational degree of freedom for manipulating said imaging probe (24) around a third axis (Rx), said driver module (26) further comprising a rotary guide (54) and rotary rail (52), said rotary rail (52) having a geometry for allowing an additional medical instrument to be positioned adjacent the imaging probe (24),
   wherein said first (Ry), second (Rz), and third (Rx) rotation axes represent a set of three Euler orientation angles and intersect at a single point remote from the remote center of motion module (14);
   the apparatus further comprising an imaging probe adapter (40) attached to the driver module (26) for securing the imaging probe (24);
   **characterised in that** the imaging probe adapter (40) is adapted to secure the imaging probe (24) such that the rotary rail (52) at least partially encircles the imaging probe (24).

2. The apparatus of claim 1, wherein at least one force sensor assembly operatively connects the driver module (26) to the adapter (40).

3. The apparatus of claim 2, wherein the at least one force sensor assembly and adapter (40) are supported by a linear rail (44) aligned longitudinally with the adapter (40).

4. The apparatus of claim 1, wherein the driver module (26) further comprises a linear rail (44) and a linear guide (46), wherein said linear rail (44) and linear guide (46) are supported by said rotary rail (52) and said rotary guide (54), wherein the linear rail (44) is actuated with a ball screw (48) by a geared servomotor (50).

5. The apparatus of claim 1, wherein said adapter (40) further comprises a needle guide.

6.  The apparatus of claim 1, further comprising a controller, wherein the controller causes the apparatus to manipulate the imaging probe (24) to sweep a region of interest using rotary motion scanning.

7.  The apparatus of claim 1, further comprising a programmable computer linked to the apparatus, wherein said programmable computer allows continuous tracking of a position of the imaging probe (24) within an available reference frame.

8.  The apparatus of claim 1, further comprising an ultrasound imaging probe as the imaging probe (24) and a computer system (124) configured to capture at least one ultrasound image and corresponding imaging probe position coordinates.

9.  The apparatus of claim 8, wherein the apparatus provides measurements of the remote center of motion and of imaging probe drive angles from which ultrasound images are acquired.

10. The apparatus of claim 8, wherein said apparatus segments information gathered from said at least one ultrasound image and uses said segments for producing a three-dimensional ultrasound image volume of features of interest in a target site.

11. The apparatus of claim 2, wherein said at least one force sensor assembly measures forces and moments exerted with respect to the imaging probe (24).

12. The apparatus of claim 1, wherein said rotary rail (52) has a horseshoe geometry.

13. The apparatus of claim 8, wherein said computer system (124) is configured to control repeated compression and retraction of the ultrasound imaging probe (24) for palpating a region of interest.


**Patentansprüche**

1.  Eine Vorrichtung zum Stützen und Manipulieren einer Bildgebungssonde (24) beim Durchführen von ultraschallgeführten Eingriffen, beinhaltend:

    ein Modul (14) mit fernem Bewegungszentrum (Remote Center of Motion), das einer Bildgebungssonde (24) mindestens einen ersten Drehungs-Freiheitsgrad um eine erste Achse (Ry) und einen zweiten Drehungs-Freiheitsgrad um eine zweite Achse (Rz) bereitstellt; und
    ein Antriebsmodul (26), das betriebsfähig mit dem Modul (14) mit fernem Bewegungszentrum verbunden ist, wobei das Antriebsmodul (26) einen linearen Freiheitsgrad zur Translation entlang einer Achse (Tx), die nach einer Längsachse der Bildgebungssonde (24) ausgerichtet ist, bereitstellt und ferner einen dritten Drehungs-Freiheitsgrad zum Manipulieren der Bildgebungssonde (24) um eine dritte Achse (Rx) bereitstellt, wobei das Antriebsmodul (26) ferner eine Drehführung (54) und eine Drehschiene (52) beinhaltet, wobei die Drehschiene (52) eine Geometrie aufweist, die ermöglichen soll, dass ein zusätzliches medizinisches Instrument neben der Bildgebungssonde (24) positioniert wird,
    wobei die erste (Ry), zweite (Rz) und dritte (Rx) Drehungsachse einen Satz von drei Euler-Orientierungswinkeln darstellen und sich in einem einzigen Punkt fern von dem Modul (14) mit fernem Bewegungszentrum schneiden;
    wobei die Vorrichtung ferner einen Bildgebungssondenadapter (40) zum Sichern der Bildgebungssonde (24) beinhaltet, der an dem Antriebsmodul (26) angebracht ist;
    **dadurch gekennzeichnet, dass** der Bildgebungssondenadapter (40) angepasst ist, um die Bildgebungssonde (24) so zu sichern, dass die Drehschiene (52) die Bildgebungssonde (24) mindestens teilweise umschließt.

2.  Vorrichtung gemäß Anspruch 1, wobei mindestens eine Kraftsensoranordnung das Antriebsmodul (26) betriebsfähig mit dem Adapter (40) verbindet.

3.  Vorrichtung gemäß Anspruch 2, wobei die mindestens eine Kraftsensoranordnung und der Adapter (40) durch eine lineare Schiene (44), die längs nach dem Adapter (40) ausgerichtet ist, gestützt werden.

4.  Vorrichtung gemäß Anspruch 1, wobei das Antriebsmodul (26) ferner eine lineare Schiene (44) und eine lineare Führung (46) beinhaltet, wobei die lineare Schiene (44) und die lineare Führung (46) durch die Drehschiene (52) und die Drehführung (54) gestützt werden, wobei die lineare Schiene (44) mit einer Kugelumlaufspindel (48) durch einen Getriebe-Servomotor (50) betätigt wird.

5.  Vorrichtung gemäß Anspruch 1, wobei der Adapter (40) ferner eine Nadelführung beinhaltet.

6.  Vorrichtung gemäß Anspruch 1, die ferner eine Steuereinheit beinhaltet, wobei die Steuereinheit bewirkt, dass die Vorrichtung die Bildgebungssonde (24) manipuliert, sodass sie unter Verwendung von Drehbewegungsscannen über einen Bereich von Interesse streicht.

7.  Vorrichtung gemäß Anspruch 1, die ferner einen mit der Vorrichtung verknüpften programmierbaren Computer beinhaltet, wobei der programmierbare Computer die kontinuierliche Verfolgung einer Position der Bildgebungssonde (24) innerhalb eines verfügbaren Bezugsrahmens ermöglicht.

8.  Vorrichtung gemäß Anspruch 1, die ferner eine Ultraschall-Bildgebungssonde als die Bildgebungssonde (24) und ein Computersystem (124), das konfiguriert ist, um mindestens ein Ultraschallbild und entsprechende Positionskoordinaten der Bildgebungssonde zu erfassen, beinhaltet.

9.  Vorrichtung gemäß Anspruch 8, wobei die Vorrichtung Messungen des fernen Bewegungszentrums und der Antriebswinkel der Bildgebungssonde bereitstellt, aus denen Ultraschallbilder erhalten werden.

10.  Vorrichtung gemäß Anspruch 8, wobei die Vorrichtung Informationen, die aus dem mindestens einen Ultraschallbild gesammelt werden, segmentiert und die Segmente verwendet, um ein dreidimensionales Ultraschallbildvolumen von Merkmalen von Interesse in einer Zielstelle zu produzieren.

11.  Vorrichtung gemäß Anspruch 2, wobei die mindestens eine Kraftsensoranordnung Kräfte und Momente, die in Bezug auf die Bildgebungssonde (24) ausgeübt werden, misst.

12.  Vorrichtung gemäß Anspruch 1, wobei die Drehschiene (52) eine Hufeisengeometrie aufweist.

13.  Vorrichtung gemäß Anspruch 8, wobei das Computersystem (124) konfiguriert ist, um das wiederholte Komprimieren und Rückziehen der Ultraschall-Bildgebungssonde (24) zum Abtasten eines Bereichs von Interesse zu steuern.

**Revendications**

1.  Un appareil pour soutenir et manipuler une sonde d'imagerie (24) lorsque des interventions guidées par ultrasons sont réalisées, comprenant :

    un module centre de mouvement distant (14) fournissant à une sonde d'imagerie (24) au moins un premier degré de liberté rotatoire autour d'un premier axe (Ry) et un deuxième degré de liberté rotatoire autour d'un deuxième axe (Rz) ; et
    un module d'entraînement (26) relié fonctionnellement au module centre de mouvement distant (14), le module d'entraînement (26) fournissant un degré de liberté linéaire pour une translation le long d'un axe (Tx) aligné avec un axe longitudinal de la sonde d'imagerie (24) et fournissant en sus un troisième degré de liberté rotatoire pour manipuler ladite sonde d'imagerie (24) autour d'un troisième axe (Rx), ledit module d'entraînement (26) comprenant en sus un guide pour rotation (54) et un rail pour rotation (52), ledit rail pour rotation (52) ayant une géométrie pour permettre à un instrument médical supplémentaire d'être positionné de façon adjacente à la sonde d'imagerie (24),
    dans lequel lesdits premier (Ry), deuxième (Rz), et troisième (Rx) axes de rotation représentent un jeu de trois angles d'orientation d'Euler et se coupent au niveau d'un point unique distant du module centre de mouvement distant (14) ;
    l'appareil comprenant en sus un adaptateur de sonde d'imagerie (40) fixé au module d'entraînement (26) pour assujettir la sonde d'imagerie (24) ;
    **caractérisé en ce que** l'adaptateur de sonde d'imagerie (40) est conçu pour assujettir la sonde d'imagerie (24) de telle sorte que le rail pour rotation (52) encercle au moins partiellement la sonde d'imagerie (24).

2.  L'appareil de la revendication 1, dans lequel au moins un ensemble de capteurs de force relie fonctionnellement le module d'entraînement (26) à l'adaptateur (40).

**3.** L'appareil de la revendication 2, dans lequel l'au moins un ensemble de capteurs de force et l'adaptateur (40) sont soutenus par un rail linéaire (44) aligné longitudinalement avec l'adaptateur (40).

**4.** L'appareil de la revendication 1, dans lequel le module d'entraînement (26) comprend en sus un rail linéaire (44) et un guide linéaire (46), lesdits rail linéaire (44) et guide linéaire (46) étant soutenus par ledit rail pour rotation (52) et ledit guide pour rotation (54), le rail linéaire (44) étant actionné avec une vis à billes (48) par un servomoteur à engrenages (50).

**5.** L'appareil de la revendication 1, dans lequel ledit adaptateur (40) comprend en sus un guide-aiguille.

**6.** L'appareil de la revendication 1, comprenant en sus un organe de commande, l'organe de commande amenant l'appareil à manipuler la sonde d'imagerie (24) afin de parcourir une région d'intérêt en utilisant un balayage par mouvement rotatif.

**7.** L'appareil de la revendication 1, comprenant en sus un ordinateur programmable connecté à l'appareil, ledit ordinateur programmable permettant un suivi continu d'une position de la sonde d'imagerie (24) à l'intérieur d'un référentiel disponible.

**8.** L'appareil de la revendication 1, comprenant en sus une sonde d'imagerie ultrasonore comme sonde d'imagerie (24) et un système informatique (124) configuré pour capturer au moins une image ultrasonore et des coordonnées de position de sonde d'imagerie correspondantes.

**9.** L'appareil de la revendication 8, l'appareil fournissant des mesures du centre de mouvement distant et d'angles d'entraînement de sonde d'imagerie auxquels des images ultrasonores sont acquises.

**10.** L'appareil de la revendication 8, ledit appareil segmentant des informations rassemblées à partir de ladite au moins une image ultrasonore et utilisant lesdits segments pour produire un volume d'image ultrasonore en trois dimensions de caractéristiques d'intérêt dans un site cible.

**11.** L'appareil de la revendication 2, dans lequel ledit au moins un ensemble de capteurs de force mesure des forces et des moments exercés en ce qui concerne la sonde d'imagerie (24).

**12.** L'appareil de la revendication 1, dans lequel ledit rail pour rotation (52) a une géométrie en fer à cheval.

**13.** L'appareil de la revendication 8, dans lequel ledit système informatique (124) est configuré pour commander une compression et une rétraction répétées de la sonde d'imagerie ultrasonore (24) pour palper une région d'intérêt.

FIG. 1

FIG. 2

a)

b)

**FIG. 3**

**FIG. 4**

FIG. 5

FIG. 6

FIG. 7

FIG. 8

FIG. 9

FIG. 10

FIG. 11

FIG. 12

FIG. 13

**FIG. 14**

**EP 2 637 571 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090234369 A1
- US 7021173 B